# EUROPEAN PATENT APPLICATION

(11) **EP 2 305 646 A1**
(43) Date of publication of application: **06.04.2011**
(21) Application number: 09382194.0
(22) Date of filing: 02.10.2009
(51) Int. Cl.: C07D 211/48, C07D 401/12

(54) **A process for the preparation of trans-(2R)-4-substituted-pipecolic acids and esters thereof, and intermediate compounds used therein**

(71) Applicant: Enantia, S.L., 08028 Barcelona (ES)
(72) Inventor: Riera Escalé, Antoni, 08028 Barcelona (ES); Comely, Alexander Christian, 08028 Barcelona (ES); Ginesta Buch, Xavier, 08028 Barcelona (ES)
(74) Representative: ZBM Patents

(57) **Abstract**

Process for the preparation of a compound of formula (III) wherein R¹ is a (C₁-C₁₀)-alkyl radical or a radical of one of the known ring systems with 1-3 rings, the rings being aromatic and being isolated or partially/totally fused and having 5-6 members, being each member independently selected from C, CH, N, NH, O, S; and the ring system being optionally substituted by one or more radicals independently selected from (C₁-C₆)-alkyl, (C₁-C₆)-alkoxy, and halogen; the process comprising: a) reacting a (C₁-C₁₀)-alkyl lithium, or a lithium salt of the ring system as defined above with a copper salt to obtain an organocuprate compound, and b) reacting the organocuprate compound obtained in step a) with di(tert-butyl) (2R)-3,6-dihydro-6-oxo-1,2(2H)-pyridinedicarboxylate_in an inert solvent system in the presence of a Lewis acid. Intermediate IV is new and is useful as an intermediate for the preparation of pharmaceutically active ingredients such as Argatroban.

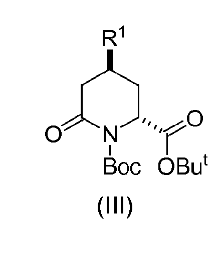

## Description

The present invention relates to a process for the preparation of trans-(2R)-4-substituted-pipecolic acids and esters thereof, to intermediate compounds used therein, and to their use in the preparation of pharmaceutically active compounds. It is also related to a process for the preparation of Argatroban.

### BACKGROUND ART

4-Alkyl or 4-aryl-pipecolic acids and their derivatives are key fragments of compounds of pharmacological interest.

Particularly, trans-(2R,4R)-4-methylpipecolic acid is a key component in the structure of antitumor agents as well as of thrombin inhibitors such as (2R,4R)-MQPA and Argatroban.

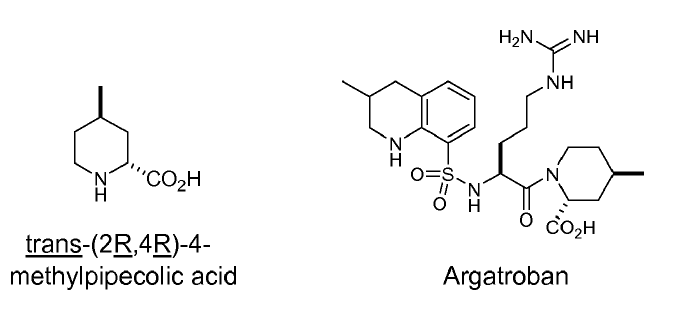

Document EP-A-8746 specifically disclose the preparation of Argatroban from the trans-(2R,4R)-4-methylpipecolic acid ethyl ester, although other possible alkyl esters are mentioned. WO 01058872 discloses the preparation of Argatroban from the trans-(2R,4R)-4-methylpipecolic acid benzyl ester.

Consequently, some trans-(2R)-4-substituted-pipecolic acids, their esters and salts thereof are, in general, important synthetic intermediates.

Several synthetic processes for trans-4-methylpipecolic acid have been disclosed, some of them not being stereoselective (giving rise either to the racemic or to a cis-trans mixture), and others involve the use of expensive catalysts (Alegret 2007³).

Hanessian 2006⁴ discloses the preparation of (2R,4R)- and (2R,4S)-4-isobutyl-6-oxopiperidine-1,2-dicarboxylic acid 1-tert-butyl ester 2-methyl ester in a 1.1:1 ratio from the 6-oxo-4,5-dehydro-D-pipecolic acid ester, i.e. without any selectivity.

Hanessian 2002⁵ discloses the stereocontrolled synthesis of 2,4-cis/trans 1-tert-butyl 2-methyl 6-oxo-4-phenyl-piperidine-1,2-dicarboxylates from 1-tert-butyl 2-methyl (2S)-3,6-dihydro-6-oxo-1,2(2H)-pyridinedicarboxylate resulting in the trans-product as the major isomer with a stereoselectivity 10:1.

Muller 1999⁶ discloses that the stereoselectivity, i.e. the cis:trans ratio, observed for the conjugate addition of organocuprates to 1-tert-butyl 2-methyl (2S)-3,6-dihydro-6-oxo-1,2(2H)-pyridinedicarboxylate depends on the structure of the transferred substituent. When the transferred substituent is methyl a low stereoselectivity in favour of the cis isomer (5:1) is obtained. No mention is made of the possible effect on stereoselectivity by the use of other esters in the 2 position.

Coe 2002⁷ discloses the preparation of mixtures of cis and trans tert-butyl (2S,4RS)-N-tert-butoxycarbonyl-4-alkenyl-6-oxopiperidine-2-carboxylate derivatives with low stereoselectivity.

Hanessian 2009⁸ discloses the preparation of some 2,4-cis di-tert-butyl 6-oxo-4-substituted-piperidine-1,2-dicarboxylates in enantiopure form. No mention of the possibility to obtain the corresponding trans diastereoisomer is made.

Therefore, from what is known in the art, it is evident that the provision of an efficient process for the preparation of certain trans-(2R)-4-substituted-pipecolic acids, their esters, and salts thereof, which would allow them to be used as intermediates in the preparation of some pharmaceutically active compounds, would be of great interest in industry.

### SUMMARY OF THE INVENTION

The present inventors have found a new process for the preparation of some trans-(2R)-4-substituted-pipecolic acids, their esters, and salts thereof. It should be emphasized that the new process obviates the use of costly catalysts, and provides the final chiral compound in a high yield and with a good chemical and enantiomeric purity. Additionally, the process provides the final chiral compound in a high trans/cis diastereoisomeric ratio.

As mentioned above, in Hanessian 2006, the preparation of (2R,4R)- and (2R,4S)-4-isobutyl-6-oxopiperidine-1,2-dicarboxylic acid 1-tert-butyl ester 2-methyl ester is carried out without any selectivity (cis:trans 1.1:1). Additionally, there is no hint therein towards the possibility of obtaining the trans-isomer with high selectivity, namely with the trans/cis diastereomeric ratio obtained by the process of the invention. Other processes related to the preparation of other esters of the corresponding enantiomer either disclose low stereoselectivities or very low yields.

Therefore, according to a first aspect of the present invention, there is provided a process for the preparation of a compound of formula (III) 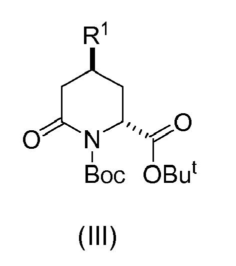 wherein R¹ is a (C₁-C₁₀)-alkyl radical, or a radical of one of the known ring systems with 1-3 rings; the rings being aromatic and being isolated or partially/totally fused and having 5-6 members, being each member independently selected from C, CH, N, NH, O, S; and the ring system being optionally substituted by one or more radicals independently selected from the group consisting of (C₁-C₆)-alkyl, (C₁-C₆)-alkoxy, and halogen; the process comprising:
a) reacting a (C₁-C₁₀)-alkyl lithium, or a lithium salt of the ring system as defined above with a copper salt to obtain an organocuprate compound; and
b) reacting the organocuprate compound obtained in step a) with a compound of formula (IV)

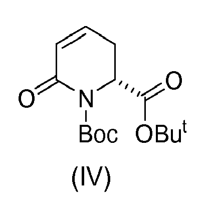

in an inert solvent system in the presence of a Lewis acid.

The presence of the tert-butyl ester in the intermediate (IV) together with the use of the mentioned organocuprate compounds seems to be the key feature giving rise to the high yield, high enantiomeric purity and high trans/cis diastereomeric ratio when the conjugate addition is carried out.

Also part of the present invention is a process as defined above, further comprising
c) reducing the amide moiety to the corresponding amine;
d) removing the N-Boc protecting group; and
e) optionally, converting the tert-butyl ester into another ester R² by transesterification or into the corresponding carboxylic acid by hydrolysis; wherein the reaction steps d) and e) can be carried out in any order,
   to obtain a compound of formula (I) or a salt thereof 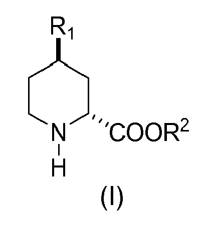 wherein R¹ is as defined above and R² is H, (C₁-C₄)-alkyl, (C₃-C₆)-cycloalkyl, or phenyl-(C₁-C₄)-alkyl, wherein the phenyl group is optionally substituted by one or more radicals independently selected from the group consisting of (C₁-C₆)-alkyl, (C₁-C₆)-alkoxy, and halogen.

As a consequence of obtaining compound of formula (III) with a trans/cis diastereoisomeric ratio of about 20:1, the final chiral compound of formula (I) also has a high diastereoisomeric ratio. Moreover, the steric hindrance of the tert-butyl ester in the compound of formula (III) helps in the quimioselective reduction of the amide.

It is worth noting that by none of the processes disclosed in the prior art the target compounds of formula (I) could be achieved, firstly, because all the prior art processes work with the opposite enantiomeric intermediates, and, secondly, because the use of methyl esters not only affords low stereoselectivities in the addition of small substituents but also would not permit the quimioselective reduction of the amide, but the methyl ester group would be also reduced.

Another aspect of the present invention relates to a process for the preparation of a pharmaceutically active compound, or a pharmaceutically acceptable salt thereof, comprising in its chemical structure an N-radical of formula (I') 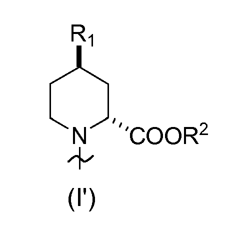 wherein R¹ and R² are as defined above, the process comprising carrying out the process for the preparation of a compound of formula (I) as above, and further comprising: f) reacting the compound of formula (I) with a compound susceptible to form an amine, amide or carbamate bond with the N of the piperazine of compound of formula (I); g) converting the obtained pharmaceutically active compound into the free acid form of the pharmaceutically active compound or into a pharmaceutically acceptable salt thereof; and h) if desired, converting the resulting free acid form of the pharmaceutically active compound into a pharmaceutically acceptable salt thereof, or converting the resulting pharmaceutically acceptable salt into their free acid form, or converting the resulting pharmaceutically acceptable salt into a different pharmaceutically acceptable salt of the pharmaceutically active compound. It is understood that, depending on the particular final pharmaceutically active compound to be obtained, the whole process can comprise additional reactions addressed to modify or add other moieties to the intermediates intervening in or obtained in step f).

Still another aspect of the present invention relates to a compound of formula (III) 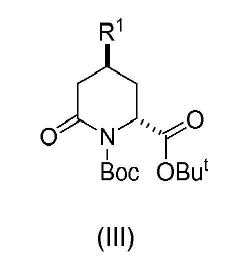 wherein R1 is as defined above.

A further aspect of the present invention relates to the use of the compound of formula (III) as defined above in the preparation of a pharmaceutically active compound, or a pharmaceutically acceptable salt thereof, comprising in its chemical structure an N-radical of formula (I') 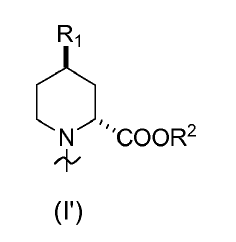 wherein R¹ and R² are as defined above.

Throughout the description and claims the word "comprise" and variations of the word, such as "comprising", are not intended to exclude other additives, components, integers or steps.

### DETAILED DESCRIPTION OF THE INVENTION

All terms used herein in this application, unless otherwise stated, shall be understood in their ordinary meaning as known in the art. Other more specific definitions for certain terms used in the present application are as set forth below and are intended to apply uniformly throughout the specification and claims unless an otherwise expressly set out definition provides a broader definition.

The term "alkyl" refers to a straight or branched saturated hydrocarbon radical. Examples of C₁-C₁₀ alkyl groups, i.e. alkyl groups having from 1 to 10 carbon atoms, include, without limitation, methyl, ethyl, n-propyl, isopropyl, n-butyl, sec-butyl, isobutyl, tert-butyl, n-pentyl, n-hexyl, n-heptyl, n-octyl, n-nonyl, and n-decyl.

The term "alkoxy" refers to radicals of the formula -OR³, where R³ is alkyl as defined above.

Illustrative non-limitative examples of suitable known ring systems include phenyl, biphenyl, 1,2,3,4-tetrahydronaphthyl, naphthalen-1-yl, 2-naphthyl, anthryl, phenanthryl, pyridyl, pyrrolyl, pyrimidinyl, furyl, thienyl, imidazolyl, oxazolyl, isoxazolyl, thiazolyl, isothiazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, 1,3,4-triazinyl, 1,2,3-triazinyl, benzofuryl, [2,3-dihydro]benzofuryl, isobenzofuryl, benzothienyl, benzotriazolyl, isobenzothienyl, indolyl, isoindolyl, 3H-indolyl, benzimidazolyl, imidazo[1,2-a]pyridyl, benzothiazolyl, benzoxazolyl, quinolizinyl, quinazolinyl, pthalazinyl, quinoxalinyl, cinnolinyl, napthyridinyl, pyrido[3,4-b]pyridyl, pyrido[3,2-b]pyridyl, pyrido[4,3-b]pyridyl, quinolyl, isoquinolyl, tetrazolyl, 5,6,7,8-tetrahydroquinolyl, 5,6,7,8-tetrahydroisoquinolyl, purinyl, pteridinyl, carbazolyl, xanthenyl or benzoquinolyl.

The term "pharmaceutically acceptable salt" of a compound means a salt that possesses the desired pharmacological activity of the parent compound. Such salts include: (i) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3- (4-hydroxybenzoyl) benzoic acid, cinnamic acid, mandelic acid, methanesulfonic acid, ethanesulfonic acid, 1,2-ethanedisulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (ii) salts formed when an acidic proton present in the parent compound either is replaced by a metal ion, such as an alkali metal ion, an alkaline earth ion, or an aluminium ion; or coordinates with an organic base such as ethanolamine, diethanolamine, triethanolamine, tromethamine, or N-methylglucamine.

As mentioned above, the conjugate addition of a suitable organocuprate compound to the compound of formula (IV) according to the process of the present invention leads to the corresponding compound of formula (III) in excellent yields and with high stereoselectivity.

The organocuprate compound is prepared by reaction of the corresponding organolithium compound, namely the corresponding (C₁-C₁₀)-alkyl lithium, or the lithium salt of a known ring system as defined above, with a copper salt, in a suitable solvent such as dimethyl ether, diethyl ether, methyl t-butyl ether, diisopropyl ether, tetrahydrofuran (THF), dioxane, and toluene. Preferably the copper salt is a copper halide, more preferably Cul.

The organolithium compound used in the process of the present invention is either a (C₁-C₁₀)-alkyl lithium, or a lithium salt of a known ring system with 1-3 rings; the rings being aromatic and being isolated or partially/totally fused and having 5-6 members, being each member independently selected from C, CH, N, NH, O, S; and the ring system being optionally substituted by one or more radicals independently selected from the group consisting of (C₁-C₆)-alkyl, (C₁-C₆)-alkoxy, and halogen.

Organolithium compounds are either commercial or they can be prepared from commercial organolithium or organohalogen compounds by metalhalogen or metal-metal exchange following standard procedures. Illustrative non-limitative examples of commercially available organolithium compounds are n-BuLi, sec-BuLi, t-BuLi, MeLi, and PhLi.

The conjugate addition reaction is carried out in a suitable solvent, and in the presence of a Lewis acid, such as a tri-(C₁-C₄)-alkylsilyl halide, BF₃·Et₂O, or Et₂AlCl. Illustrative non-limitative examples of suitable solvents are dimethyl ether, diethyl ether, methyl t-butyl ether, diisopropyl ether, tetrahydrofuran (THF), dioxane, and toluene. Especially good results are obtained when the conjugate addition reaction is carried out in the presence of trimethylsilyl chloride (TMSCI).

The temperature at which the conjugate addition is carried out depends on the organocuprate compound used. Generally, the reaction is carried out at a temperature comprised between 0 °C and -78 °C. Preferably, the reaction is carried out in THF at about -78 °C.

In a preferred embodiment of the process of the invention, compounds of formula (III) are those where R¹ is a (C₁-C₁₀)-alkyl radical. Preferably, R¹ is methyl, ethyl, n-propyl, isopropyl, n-butyl, isobutyl, sec-butyl, or tert-butyl. More preferably, R¹ is methyl.

In another preferred embodiment of the process, compounds of formula (III) are those where R¹ is phenyl, or phenyl substituted by one or more radicals independently selected from the group consisting of (C₁-C₆)-alkyl, (C₁-C₆)-alkoxy, and halogen.

As mentioned above, also part of the present invention is a process as defined above, further comprising
c) reducing the amide moiety to the corresponding amine;
d) removing the N-Boc protecting group; and
e) optionally, converting the tert-butyl ester into another ester R² by transesterification or into the corresponding carboxylic acid by hydrolysis; wherein the reaction steps d) and e) can be carried out in any order,
   to obtain a compound of formula (I) or a salt thereof 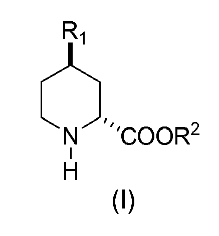 wherein R¹ and R² are as defined above. It is considered that also part of the invention is a process comprising either step c) or steps c) and e) to yield the corresponding intermediate compounds of formula (II) 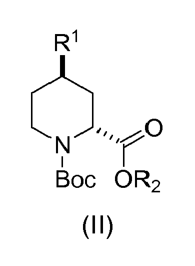 wherein R¹ and R² are as defined above.

The reduction of the amide moiety of the compound of formula (III) to the corresponding amine can be carried out by reaction with a borane reagent such as BH₃·THF or BH₃·SMe₂, or other borane sources, such as NaBH₄/I₂, or other boron reducing agents, such as LiBHEt₃, or aluminium reducing agents, such as AIHBuⁱ₂.

The deprotection reaction, i.e the removal of the N-Boc protecting group, can be carried out by conventional methods already known by those skilled in the art. By way of example, deprotection can be carried out by treatment in an acid medium, such as in hydrochloric acid or trifluoroacetic acid. Preferably, the deprotection is carried out in HCl in dioxane. Removal of the N-Boc protecting group can be carried out selectively or in a single step together with the tert-butyl ester hydrolysis step.

The tert-butyl ester moiety can be transformed easily into a carboxylic acid or another ester by hydrolysis or transesterification following standard procedures, namely by reaction with an acid or and alcohol, preferably in the presence of an acid or base as a catalyst.

Scheme I depicts a particular embodiment of the preparation of the compound of formula (I) wherein R¹ is methyl and R² is tert-butyl, using as starting material N-Boc-D-aspartic-(O^{t}Bu) acid, via the corresponding intermediate of formula (III).

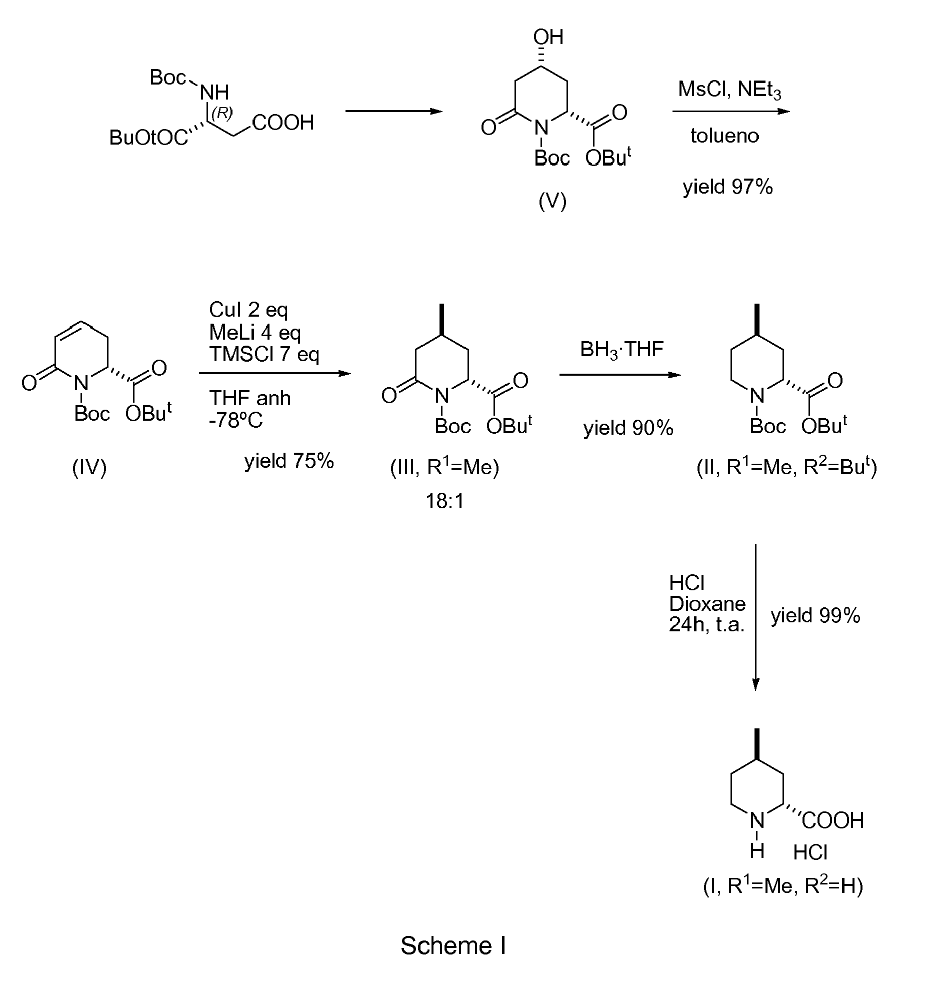

The compound of formula (V) in Scheme I, namely di(tert-butyl) (2R,4R)-4-hydroxy-6-oxo-1,2-piperidinedicarboxylate, can be prepared according to the process disclosed in Chaloin 2008⁹, but starting from tert-butyl N-Boc-D-aspartate.

As is illustrated in Scheme I, the compound of formula (IV) can be prepared from the compound of formula (V) by reaction with MsCl in the presence of triethylamine. The reaction could also be carried out with a reagent suitable to convert a hydroxyl group to a leaving group, such as a sulfonyl chloride compound of formula CISO₂R₄, where R₄ is (C₁-C₄)-alkyl, phenyl or phenyl mono- or disubstituted by a (C₁-C₄)-alkyl radical. Preferably, the sulfonyl chloride compound may be selected from benzenesulfonyl chloride, p-toluenesulfonyl chloride (TsCl), and methanesulfonyl chloride (MsCl). Triethylamine could also be replaced by any other organic tertiary amine.

The compounds obtained by the chemical transformations of the present invention can be used for the following steps without further purification or can be effectively separated and purified by employing a conventional method well known to those skilled in the art, such as recrystallization, column chromatography, or by transformation into a salt or by washing with an organic solvent or with an aqueous solution, eventually adjusting the pH.

As mentioned above, the compound of formula (III) of the invention is useful as an intermediate for the preparation of pharmaceutically active compounds comprising in their chemical structure an N-radical of formula (I') 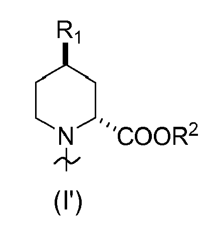 wherein R¹ and R² are as defined above.

For the preparation of the pharmaceutically active compounds comprising in their chemical structure an N-radical of formula (I') as defined above, various methods can be employed depending upon the particular starting materials and/or intermediates involved.

In a preferred embodiment, the pharmaceutically active compound is Argatroban. Illustrative non-limitative examples of the preparation of Argatroban from the compound of formula (I) are disclosed in the Schemes of pages 3, 4 and 6 of EP-A-8746, and in Scheme 1 of WO 01058872.

Furthermore, the present invention covers all possible combinations of particular and preferred groups described hereinabove.

Additional objects, advantages and novel features of the invention will be set forth in part in the description, and in part will become apparent to those skilled in the art upon examination of the description or may be learned by practice of the invention. The following examples and drawings are provided by way of illustration, and are not intended to be limiting of the present invention.

### EXAMPLES

### EXAMPLE 1. Di(tert-butyl) (2R)-3,6-dihydro-6-oxo-1,2(2H)-pyridinedicarboxylate (IV)

To a suspension of di(tert-butyl) (2R,4R)-4-hydroxy-6-oxo-1,2-piperidinedicarboxylate (V) (13.5 g, 42.8 mmol) in toluene (300 mL) under inert atmosphere, triethylamine (18 mL, 129.3 mmol) is added. The mixture is cooled to 0 °C and MsCl (4.0 mL, 58.3 mmol) is added dropwise. Stirring is maintained at room temperature for 1.5 h and the solution is washed with sat. NaHCO₃ (150 mL) and with 10% NaCl (2x100 mL). The organic phase is dried over MgSO₄ and is filtered and the solvent is distilled off to obtain the title compound as a white solid (12.3 g, 97%).

### EXAMPLE 2. Di(tert-butyl) (2R,4R)-4-methyl-6-oxo-1,2-piperidinedicarboxylate (III, R¹=Me)

To a suspension of Cul (25.6 g, 134.5 mmol) in THF (800 mL) at -78 °C under inert atmosphere a 3 M solution of MeLi in diethoxymethane (90 mL, 269 mmol) is added until a clear yellow solution is obtained. If necessary, stirring is maintained for some minutes while increasing the temperature to 0 °C. The solution is cooled again to -78 °C. To this solution of lithium dimethylcuprate, TMSCI (30 mL, 235 mmol) is added.

In another flask, a solution of di(tert-butyl) (2R)-3,6-dihydro-6-oxo-1,2(2H)-pyridinedicarboxylate (IV) (20 g, 67 mmol) in THF (400 mL) is prepared and cooled to -78 °C. TMSCI (30 mL, 235 mmol) is added. This solution is added to the cuprate solution previously obtained at -78 °C through a cannula. Stirring is maintained at -78 °C for 2 h and the mixture is warmed to room temperature. A 10% NH₄OH solution in sat.NH₄Cl is added, the phases are separated and extraction of the aqueous layer with ethyl acetate is carried out. The organic phase is subsequently washed with the same aqueous solution (3x400 mL). The organic phase is dried over MgSO₄ and the solvent is distilled off to obtain the title compound as a pale yellow solid containing about 5% of the cis isomer (16 g, 75%): ¹H NMR (200 MHz, CDCl₃) δ 4.57 (dd, *J* = 5.6, 2.6 Hz, 1H), 1.51 (s, 9H), 1.47 (s, 1H), 1.99 (d, J = 6.2 Hz, 3H).

### EXAMPLE 3. Di(tert-butyl) (2R,4R)-4-methyl-1,2-piperidinedicarboxylate (II, R¹=Me)

To a solution of di(tert-butyl) (2R,4R)-4-methyl-6-oxo-1,2-piperidinedicarboxylate (III) (15 g, 49.4 mmol) in THF (100 mL) at 0 °C under inert atmosphere a 1 M solution of BH₃.THF in THF (100 mL, 100 mmol) is added. Stirring is maintained at room temperature for 2.5 h, the mixture is cooled to 0 °C and MeOH (10 mL) is added. The solvents are distilled off and the residue is redissolved in AcOEt (150 mL). The solution is washed with sat. NaHCO₃ (80 mL), 2M KHSO₄ (80 mL) and sat. NaCl (80 mL). The organic phase is dried over MgSO₄ and the solvent is distilled off to obtain the title compound as a transparent oil (13 g, 90%): ¹H NMR (400 MHz, CDCl₃) δ 4.78 (d, *J* = 5.2 Hz, 1H rotamer A), 4.58 (d, *J* = 4.8 Hz, 1H rotamer B), 4.00 (dd, *J* = 12.8, 3.2 Hz, 1H B), 3.91 (dd, *J* = 12.8, 4.0 Hz, 1H A), 3.01-2.85 (m, 1H A + 1H B), 2.18-2.10 (m, 1H A + 1H B), 1.66-1.59 (m, 1H A + 1H B), 1.46 (s, 9H A + 9H B), 1.45 (s, 9H A), 1.44 (s, 9H B), 1.31-0.98 (m, 3H A + 3H B), 0.92 (d, *J* = 6.4 Hz, 3H A + 3H B).

### EXAMPLE 4. (2R,4R)-4-Methyl-2-piperidinecarboxylic acid chlorohydrate or (2R-trans)-4-methylpipecolic acid chlorohydrate (I, R¹=Me, R²=H)

Di(tert-butyl) (2R,4R)-4-methyl-1,2-piperidinedicarboxylate (II) (2.48 g, 8.29 mmol) is dissolved in a 4M HCl solution in dioxane (14.5 mL, 58.0 mmol) and stirring is maintained at room temperature for 24 h. The solvent is distilled off to obtain the title compound as a white solid in quantitative yield: ¹H NMR (400 MHz, CD₃OD) δ 4.27 (dd, *J* = 5.2, 5.2 Hz, 1H), 3.29-3.26 (m, 2H), 2.24-2.19 (m, 1H), 1.92-1.80 (m, 2H), 1.80-1.71 (m, 2H), 1.06 (d, *J* = 6.4 Hz, 3H).

### REFERENCES

1. EP-A-8746
2. WO 01058872
3. C. Alegret et al., "Enantioselective Synthesis of trans-4-Methylpipecolic Acid", J. Org. Chem. 2007, vol. 72, pp. 7688-7692
4. S. Hanessian et al. "Design and Synthesis of Diversely Substituted Azacyclic Inhibitors of Endothelin Converting Enzyme", J. Org. Chem. 2006, vol. 71, pp. 2760-2778
5. S. Hanessian et al. "Stereocontrolled synthesis of a prototype library of enantiopure 2,4-disubstituted 4-aryl-6-piperidinones and piperidines" Tetrahedron Lett. 2002
6. M. Muller et al. "The Synthesis of α-Amino-γ-substituted Adipic Acids: Stereoelectronic Effects During the 1,4-Addition of Organocuprates to Methyl N-tert-butoxy-6-oxo-1,2,3,6-tetrahydropyridine-2-carboxylate", Chem. Commun. 1999, pp. 683-684
7. D. Coe et al. "Extension of the "Ring Switch" Approach to Glutamate Antagonists to δ-Lactam Urethanes" J. Chem. Soc., Perkin Trans. 1, 2002, pp. 2459-2472
8. S. Hanessian et al. "Diastereoselective Synthesis of Functionally Diverse Substituted Pipecolic Acids" Synlett 2009, No. 1, pp. 71-74
9. Chaloin et al. "Synthesis of Enantiopure Di(tert-butyl) (2S,4S)-4-hydroxy-6-oxo-1,2-piperidinedicarbox-ylate. A Useful Building Block for the Preparation of 4-Hydroxypipecolate Derivatives" Organic Syntheses, 2008, vol. 85, pp. 147-157

## Claims

1. A process for the preparation of a compound of formula (III) 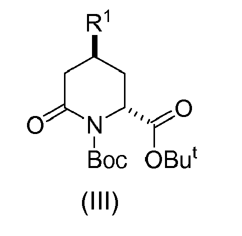 wherein R¹ is a (C₁-C₁₀)-alkyl radical, or a radical of one of the known ring systems with 1-3 rings; the rings being aromatic and being isolated or partially/totally fused and having 5-6 members, being each member independently selected from C, CH, N, NH, O, S; and the ring system being optionally substituted by one or more radicals independently selected from the group consisting of (C₁-C₆)-alkyl, (C₁-C₆)-alkoxy, and halogen;
the process comprising:
a) reacting a (C₁-C₁₀)-alkyl lithium, or a lithium salt of the ring system as defined above with a copper salt to obtain an organocuprate compound, and
b) reacting the organocuprate compound obtained in step a) with a compound
of formula (IV) 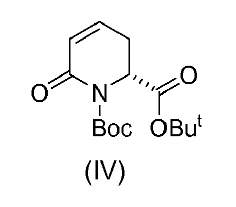 in an inert solvent system in the presence of a Lewis acid.

2. The process according to claim 1, wherein R¹ is a (C₁-C₁₀)-alkyl radical.

3. The process according to claim 2, wherein R¹ is methyl.

4. The process according to claim 1, wherein R¹ is phenyl, or phenyl substituted by one or more radicals independently selected from the group consisting of (C₁-C₆)-alkyl, (C₁-C₆)-alkoxy, and halogen.

5. The process according to any one of claims 1 to 4, wherein step b) is carried out in the presence of trimethylsilyl chloride.

6. The process according to any one of claims 1 to 5, further comprising c) reducing the amide moiety to the corresponding amine;
d) removing the N-Boc protecting group; and
e) optionally, converting the tert-butyl ester into another ester by transesterification or into the corresponding carboxylic acid by hydrolysis; wherein the reaction steps d) and e) can be carried out in any order,
to obtain a compound of formula (I) or a salt thereof 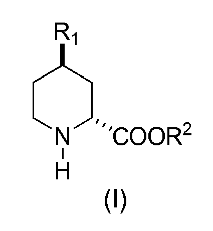 wherein R¹ is as defined in any one of claims 1 to 4, and R² is H, (C₁-C₄)-alkyl, (C₃-C₆)-cycloalkyl, or phenyl-(C₁-C₄)-alkyl, wherein the phenyl group is optionally substituted by one or more radicals independently selected from the group consisting of (C₁-C₆)-alkyl, (C₁-C₆)-alkoxy, and halogen.

7. A process for the preparation of a pharmaceutically active compound, or a pharmaceutically acceptable salt thereof, comprising in its chemical structure an N-radical of formula (I') 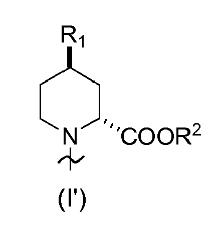 wherein R¹ is as defined in any one of claims 1 to 4, and R² is is as defined in claim 6,
the process comprising carrying out the process as defined in claim 6,
and further comprising:
f) reacting the compound of formula (I) with a compound susceptible to forming an amine, amide or carbamate bond with the N of the piperazine of the compound of formula (I),
g) converting the obtained pharmaceutically active compound into the free acid form of the pharmaceutically active compound or into a pharmaceutically acceptable salt thereof;
and
h) if desired, converting the resulting free acid form of the pharmaceutically active compound into a pharmaceutically acceptable salt thereof, or converting the resulting pharmaceutically acceptable salt into its free acid form, or converting the resulting pharmaceutically acceptable salt into a different pharmaceutically acceptable salt of the pharmaceutically active compound.

8. The process according to claim 7, wherein the pharmaceutically active compound is Argatroban.

9. A compound of formula (III) 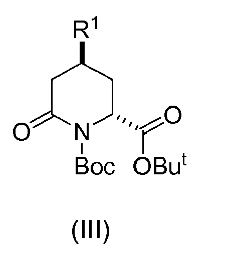 wherein R¹ is as defined in any one of claims 1 to 4.

10. The compound according to claim 9, wherein R¹ a (C₁-C₁₀)-alkyl radical.

11. The compound according to claim 9, wherein R¹ is methyl.

12. The compound according to claim 9, wherein R¹ is phenyl, or phenyl substituted by one or more by one or more radicals independently selected from the group consisting of (C₁-C₆)-alkyl, (C₁-C₆)-alkoxy, and halogen.

13. Use of the compound of formula (III) as defined in claim 9 in the preparation of a pharmaceutically active compound, or a pharmaceutically acceptable salt thereof, comprising in its chemical structure an N-radical of formula (I') 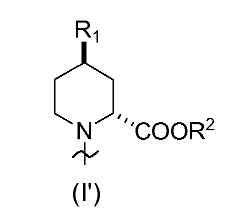 wherein R¹ is as defined in any one of claims 9 to 12, and R² is as defined in claim 6.
